# EUROPEAN PATENT SPECIFICATION

(11) **EP 4 545 028 B1**
(45) Date of publication and mention of the grant of the patent: **28.10.2026**
(21) Application number: 25150585.5
(22) Date of filing: 24.11.2020
(51) Int. Cl.: A61B 18/14, A61B 18/00

(54) **EUS ACCESS DEVICE WITH ELECTROSURGERY-ENHANCED PUNCTURE**
EUS-ZUGANGSVORRICHTUNG MIT ELEKTROCHIRURGIEVERSTÄRKTER PUNKTION
DISPOSITIF D'ACCÈS À DES EUS À PERFORATION AMÉLIORÉE PAR ÉLECTROCHIRURGIE

(30) Priority: 24.12.2019 US 201962953307 P
(43) Date of publication of application: 30.04.2025
(62) Document number(s) of the earlier application(s) in accordance with Art. 76 EPC: 20828881.1 / 3 986 308
(73) Proprietor: Boston Scientific Scimed, Inc., Maple Grove, MN 55311 (US)
(72) Inventor: SCOTT, Serena, Worcester, Massachusetts 01606 (US); BENNING, Christopher A., Hopkinton, Massachusetts 01748 (US); CALLAGHAN, David, Ashland, Massachusetts 01721 (US); HANSEN, Katrina, Shrewsbury, Massachusetts 01545 (US)
(74) Representative: Vossius & Partner Patentanwälte Rechtsanwälte mbB

(56) References cited:
- US-A- 5 403 311
- US-A1- 2013 211 176
- US-A1- 2019 374 281
- US-B1- 6 200 313
- US-B1- 7 077 842

## Description

### Field

The present disclosure relates to endoscopic ultrasound (EUS) access devices for accessing anatomical structures e.g. the pancreatico-biliary tree.

### Background

Endoscopic ultrasound (EUS) access procedures, e.g., under ultrasound guidance, may be used to access anatomical structures such as the pancreatico-biliary tree and pancreatic pseudocysts. A pancreatico-biliary access procedure, such as a procedure to insert a stent and bypass a blockage, may differ from other types of access procedures in that the target anatomy is exceedingly narrow. Many EUS access devices lack the maneuverability for biliary procedures and, even when possessing sufficient maneuverability, may encounter other difficulties. For example, devices with long, thin sharps may easily make an initial puncture hole, but risk extending the sharp too far, e.g. until the other side of the bile duct or other non-targeted tissue is penetrated.

Additionally, if an access cannula rides along the outer diameter of the sharp during the initial puncture, a long sharp may not bring the access cannula far enough distally into the puncture hole to maintain access to the duct after the sharp is removed. In another example, devices with shorter, thicker sharps may pose less of a risk for unintended punctures, but may make the initial puncture more difficult, e.g., by increasing the force required to puncture the target tissue. In still another example, a blunt access cannula may fail to follow the sharp tip into the puncture hole. US 2013/211176 A1 discloses an apparatus for causing tissue ablation at a specified therapeutic site in the body of a patient. The apparatus comprises an ablation device having a distal end and a proximal end and a central lumen extending along its length, the distal end comprising at least one energy delivery element suitable for causing tissue ablation. A penetrating member having a distal end and a proximal end, the distal end comprising a sharp tip suitable for piercing tissue and creating a channel for the device in the tissue, is coaxially positioned within the central lumen of the ablation device and is capable of being advanced distally out of the central lumen of the device and retracted back to within the central lumen of the device. US 6 200 313 B1 discloses a puncture instrument. US 7 077 842 B1 discloses a device for ablation of target tissue in the living body. US 2019/374281 A1 discloses an electrosurgical device for puncturing tissue. US 5 403 311 A discloses a catheter which includes a flexible elongated catheter body that defines a needle-receiving, or probe-receiving, lumen, a retractable tissue-penetrable needle, or probe, and an electrode mounted on the distal portion of the catheter body.

### Summary

The invention is defined by the features of claim 1. Preferred embodiments are defined by the features of the dependent claims.

### Brief Description

Figs. 1a-1b show the distal end of an endoscopic access device.
Fig. 2a shows an endoscopic access device with a long sharp.
Fig. 2b shows an endoscopic access device with a short sharp.
Fig. 2c shows an endoscopic access device with an excessive displacement formed between the sharp and the J-tip.
Fig. 3 shows a distal tip of a microcatheter having an exposed metal end that may be energized for hot puncturing in an EUS access procedure.
Fig. 4 shows a sharp having an exposed metal end that may be energized for hot puncturing in an EUS access procedure.
Fig. 5 shows a handle for controlling an EUS access procedure.
Fig. 6 shows a fine needle aspiration (FNA) needle having a blunted end that may be energized for hot puncturing in an EUS access procedure.

### Detailed Description

The present disclosure may be further understood with reference to the following description and the appended drawings, wherein like elements are referred to with the same reference numerals. The exemplary embodiments describe EUS access devices with a hot sharp and/or J-tip for effective puncturing and placement of the J-tip with low puncture forces and lower risk of losing access to the target tissue. In the present disclosure, the term "hot" refers to an element that is electrosurgically activated. Hot elements are designed so that, when activated, they pass a current through tissue, while remaining relatively unheated as they run a current through the target tissue so that electrical resistance of the target tissue causes the target tissue to heat and boil, thus enabling the target tissue to be cut and dilated. In the present disclosure, the term "cold" refers to an element that is not electrosurgically activated.

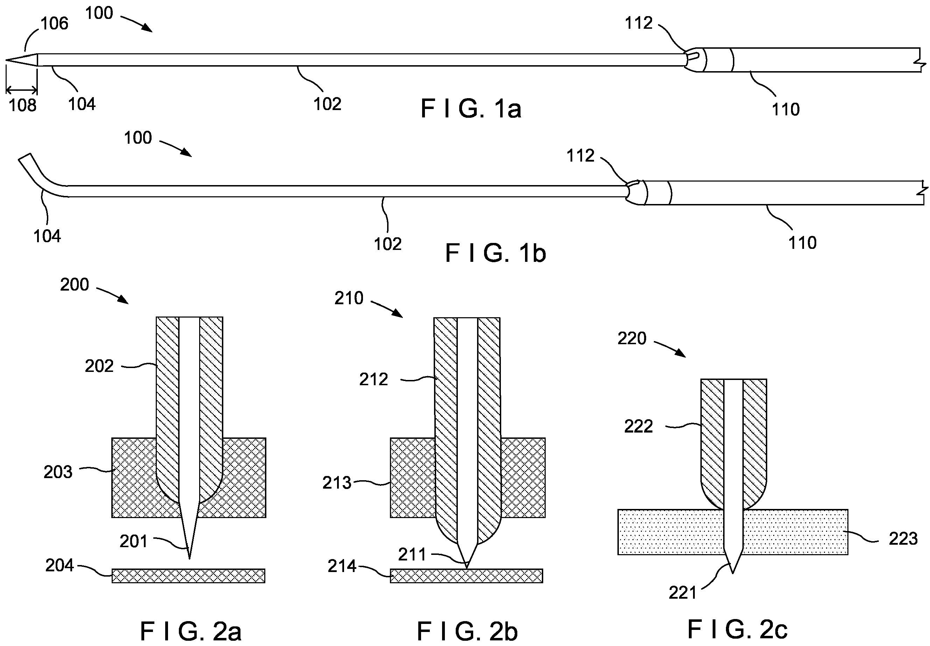

Figs. 1a-1b show an exemplary EUS access device 100 including a microcatheter 102 (i.e., access cannula) with a flexible distal tip 104 biased to assume, when unconstrained, a natural J-shape (J-tip). A puncturing element 106 with a pointed tip (i.e., the sharp) is advanced through the lumen of the microcatheter 102 so that the flexible J-tip 104 is straightened via the stiffness of the sharp 106 until the sharp 106 extends distally out the distal end of the J-tip 104 by a desired distance so that the sharp 106 may be used to puncture target tissue and the sharp 106 and J-tip 104 may be advanced together into the target anatomy.

The distance 108 by which the distal tip of the sharp 106 protrudes distally beyond the distal end of the J-tip 104 when the sharp 106 is inserted therein is referred to as the "setback." After the J-tip 104 and sharp 106 have been advanced into the target anatomy as desired, the sharp 106 is withdrawn proximally out of the J-tip 104 freeing the J-tip 104 to return to its curved J-shape, as shown in Fig. 1b. A guidewire may then be inserted through the lumen of the microcatheter 102 and out of the distal end of the J-tip 104 into the target anatomy. Before, while, or after the guidewire is inserted into the microcatheter 102, the J-tip 104 may be rotated to point a distal opening of the J-tip 104 in a desired direction within the target anatomy.

For example, where the target anatomy is a bile duct, the J-tip 104 may be rotated so that the distal opening of the lumen of the microcatheter 102 faces either upstream in the bile duct or downstream toward an outlet of the bile duct into the small intestine. When the J-tip 104 is oriented as desired, the guidewire is passed through the microcatheter 102 to exit the opening at the distal end of the J-tip 104 and is extended out of the J-tip 104 in the desired direction along the bile duct by a desired distance. At this point, a flexible electrosurgical sheath 110 with an electrosurgical tip 112 may be advanced over the microcatheter 102 and the J-tip 104 to dilate a hole by which the microcatheter 102 exited the small intestine and a hole by which the microcatheter 102 entered the target bile duct. The electrosurgical tip may dilate the access holes(s) (fistula) to e.g. 5-11 Fr. As would be understood by those skilled in the art, an electrode of the electrosurgical tip may be activated when this tip is located at the entrance to and within hole to cut and widen the holes to facilitate access to the target anatomy for further procedures (e.g., placing a stent therein to bypass a blockage).

The sharp 106 is typically used to make a starter hole in the anatomy through which the wider diameter J-tip 104 may be pushed, so that when the sharp 106 is removed the J-tip 104 is firmly inserted through the puncture hole within the target anatomy. However, various complications may arise when performing this operation.

For example, an access device 200 with a long, thin sharp 201, as shown in Fig. 2a, may puncture the tissue 203 easily, i.e., reduce puncture forces necessary to make the hole. However, the long setback between the distal tip of the sharp 201 and the distal end of the J-tip 202 requires the sharp to be pushed further into the tissue 203 before the J-tip 202 is firmly inserted in the puncture hole. Thus, using a long, thin sharp 201 risks pushing the sharp 201 too far distally and inadvertently puncturing tissue 204 distal to the initially punctured tissue 203. Conversely, if the long sharp 201 is not pushed far enough to firmly entrench the J-tip 202, the J-tip 202 may recede from and fall out of the puncture hole when the sharp 201 is removed.

In another example, an access device 210 with a shorter, thicker sharp 211, as shown in Fig. 2b, may carry less risk of unintentionally puncturing the tissue 214 distal to the initially punctured tissue 213, or having the J-tip 212 fall out of the puncture hole. However, the force required to puncture target tissue with a device including such a thickened sharp 211 are increased as compared to those required with a long, thin sharp.

In still another example, an access device 220, as shown in Fig. 2c, may have a J-tip 222 that catches on the anatomy walls as the sharp 221 is advanced through the tissue 223, resulting in a displacement between the sharp tip 221 and the J-tip 222 and potentially pushing the tissue 223 distally as the J-tip 222 is forced against the tissue wall (i.e. tenting). Tenting may occur when a blunt or poorly tapered J-tip is used. However, using a sharper-edged J-tip may risk damaging surrounding tissue when the J-tip is rotated within the duct to direct the guidewire.

The exemplary embodiments describe EUS access devices with one or more energized features for hot puncturing that address the aforementioned issues. An electrosurgical generator, such as an Erbe generator, may be used to apply an RF cutting current to the device to allow the puncturing tip(s) to pass easily into the tissue with low puncture forces. The devices may be substantially similar to the device described in Figs. 1a-1b, with modifications to be described below.

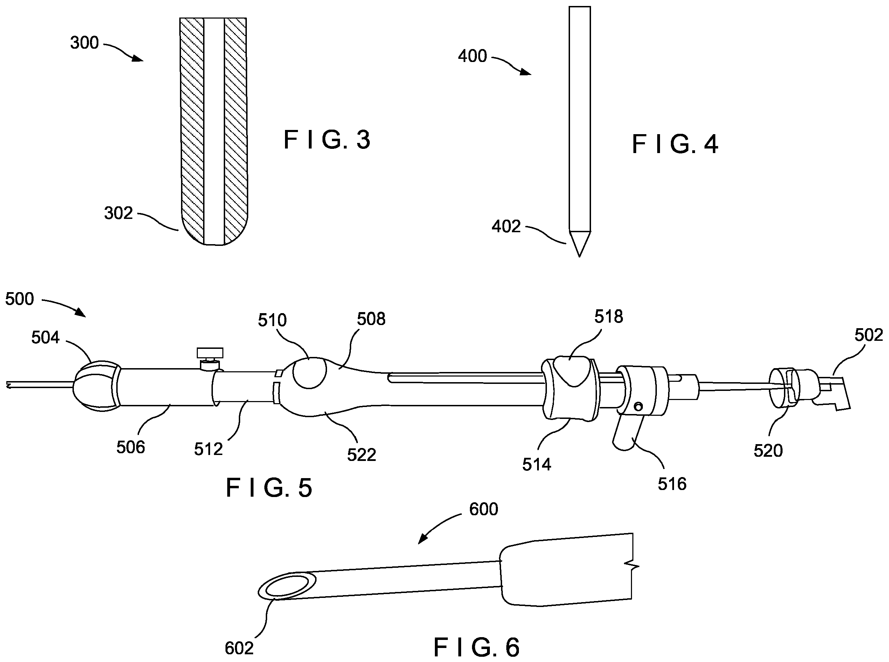

Fig. 3 shows a distal tip 300 (J-tip) of a microcatheter (in its straightened configuration) having an exposed metal end 302 that may be energized for hot puncturing in an EUS access procedure. The metal end 302 is blunt to minimize trauma to the surrounding tissue as the J-tip 300 (in its curved configuration) is rotated within a duct to direct a guidewire as desired. Any sharp may be used with the distal tip 300 as would be understood by those skilled in the art. However, a shorter sharp may be used without increasing the required puncture forces. As noted above, the use of a cold, blunt J-tip with a short sharp requires increased puncture forces. However, a hot, blunt J-tip has been shown to puncture with relatively low puncture forces. In this embodiment, the hot, blunt J-tip may have a minimum setback of approximately 0.5 mm.

When the microcatheter is entirely metal, the entirety of the microcatheter with the exception of the exposed metal end 302 is insulated to prevent the heating or cutting of non-targeted tissue (i.e., tissue other than that at the target site). The exposed metal end 302 may, for example, be less than ~1mm long to minimize thermal damage to the target site during the cutting. The exposed metal end 302 of the hot J-tip 300 may also be wired to the handle, and the remainder of the microcatheter may be formed of a different, non-conductive material eliminating the need for a separate electrically insulative coating.

Fig. 4 shows a sharp 400 having an exposed metal end 402 that may be energized for hot puncturing in an EUS access procedure. The exposed metal end 402 may be a short, sharpened tip, or may be relatively blunt. When the metal end 402 is sharpened the sharp 400 may be used for cold puncture as well. However, if the attempted cold puncture is unsuccessful, a user has the option to energize the exposed metal end 402 for a hot puncture.

Similarly to the hot J-tip 300, the hot sharp 400 may have a flexible shaft of the same conductive material (e.g. metal) as the exposed metal end 402 yet be insulated along its length (with the exception of the exposed metal end 402). If the access cannula being used with the sharp 400 is not intended to have a hot J-tip, the J-tip may be insulated to prevent conduction of electricity from the sharp 400 through the J-tip. Alternately, the J-tip may not be insulated and the access device will have both a hot sharp and a hot J-tip. In such a case, only one of the J-tip or the sharp need be connected to the electrosurgical generator (by wire, or otherwise) to effectively energize both tips.

Although a hot sharp cuts well enough to allow a blunt, cold J-tip to pass through the fistula, energizing both of the tips may further reduce puncture forces. However, choosing which one or both of the J-tip and sharp to energize may depend on the nature of the intervention. For example, a hot J-tip has been shown to make a larger fistula than a cold J-tip. A larger fistula would not affect a procedure such as a stent implant, considering the fistula will need to be made larger still with an electrosurgical dilator prior to implanting the stent. However, for a procedure where a smaller hole is desired (e.g., a rendezvous procedure), an energized sharp with a cold J-tip may be preferred over energizing both the sharp and the J-tip.

In an alternate embodiment, a hot blunt stylet may be used instead of a sharp. The stylet has a blunt distal end, as opposed to the sharp which has a sharpened distal end. In this embodiment, the stylet and the J-tip may have a very short setback, e.g. 2 mm, to ensure that when the stylet tip is passed into the duct the access cannula will pass into the duct as well. This embodiment allows for targeting of smaller tissues as there is lower risk of the tip extending to the opposite side of the duct and inadvertently puncturing the opposite side.

The aforementioned embodiments may be implemented via an endoscope, with the device having a handle for controlling the endoscopic procedure.

Fig. 5 shows a handle 500 for controlling an EUS access procedure. The handle 500 extends from a proximal sharp hub 502 to a distal collar 504 that attaches to a coupling at a proximal end of an endoscope shaft. The sharp hub 502 is connected to the sharp such that the sharp hub 502 can be pulled to withdraw the sharp from the device. The sharp hub 502 includes a generator connection 520 at which a source of electrical energy may be coupled to the device. The handle 500 includes a length adjust 506 via which a user can adjust a length of the handle 500 so that, when coupled to an endoscope, a length of the electrosurgical sheath will extend to a desired distance distally beyond a distal end of the endoscope (i.e., the length adjust may be used to achieve an extension of the device out the endoscope). The handle 500 further includes a puncture actuator 508 that is slidable over a base 512 of the handle 500 so that, when unlocked via a puncture actuator lock 510, the J-tip and the sharp are advanced distally out of a sheath (e.g., electrosurgical sheath 110 shown in Fig. 1) so that the J-tip and the sharp can penetrate target tissue to a desired depth. The puncture actuator 508 includes a generator connection 522 at which a source of electrical energy may be coupled to the device.

The handle 500 further includes an electrosurgical sled 514 slidably mounted over the puncture actuator 508 so that the sheath can be advanced distally over the J-tip to bring an electrosurgical tip (e.g., electrosurgical tip 112 shown in Fig. 1) at the distal end of the sheath into contact with target tissue so that the tissue may be treated by the application of energy from the tip (e.g., to cut and dilate tissue around an opening formed through a wall of the gastrointestinal tract and an entry opening into a target pancreatico-biliary lumen). The electrosurgical sled 514 includes a generator connection 516 at which a source of electrical energy may be coupled to the device. The electrosurgical sled 514 is maintained in a desired position over the puncture actuator 508 via an electrosurgical sled lock 518. Similarly to the puncture actuator lock 510, the electrosurgical sled lock 518 includes a projection that may engage a geared surface on the base 512 until the corresponding lock is depressed to disengage the connection with the base 512, permitting the puncture actuator 508 or the electrosurgical sled 514 to slide over the base 512.

The generator connection 516 provides the coupling to electrical energy that may be transmitted to the electrosurgical sled 514. The generator connections 520 and 522 would provide energy to each of the metal end 402 of the sharp and the metal end 302 of the J-tip microcatheter, respectively, in the manner described above. The base 512 may have depth indicators thereon, providing greater control of the puncture depth of the sharp/J-tip and the electrosurgical sheath length. As indicated previously, the ideal depth of the puncture may vary for different procedures and for different sharp/J-tip configurations. Thus, the handle 500 has precise puncture depth controllability compatible with different access devices having different puncture depths.

Fig. 6 shows a fine needle aspiration (FNA) needle 600 having a blunted end 602 that may be energized for hot puncturing in an EUS access procedure. The FNA needle 600 has a lumen for a guidewire to extend through. A blunted, as opposed to sharp, needle allows for easier guidewire directionality with less risk of skiving the guidewire. The blunted end 602, despite being blunt, is able to puncture anatomy when energized.

Each of the aforementioned energized puncturing features may be actuated via an electrosurgical plug extending from or built into the endoscope handle. The hot tip feature may be actuated at any time during the endoscopic intervention. Thus, a clinician may begin a procedure using e.g. a cold sharp tip and, if the cold puncture is unsuccessful, actuate the hot tip and perform a hot puncture.

It will be appreciated by those skilled in the art that changes may be made to the embodiments described above without departing from the invention as defined by the appended claims. It should further be appreciated that structural features and methods associated with one of the embodiments can be incorporated into other embodiments. It is understood, therefore, that this invention is not limited to the particular embodiment disclosed, but rather modifications are also covered within the scope of the present invention as defined by the appended claims.

## Claims

1. An access device (100; 200; 210; 220), comprising:
a catheter (102) including a lumen extending therethrough, the catheter being sized and shaped to extend through an endoscopic shaft to a target tissue within a living body, the catheter (102) including a distal end (104; 202; 212; 222; 300) biased to assume a J-shaped curve relative to a portion of the catheter (102) extending proximally from the distal end (104; 202; 212; 222; 300), when the distal end (104; 202; 212; 222; 300) is unconstrained, wherein the distal end (104; 202; 212; 222; 300) is blunt; and
a puncturing device (106; 201; 211; 221; 400) sized and shaped to extend through the lumen of the catheter (102) and configured to extend distally out of the distal end (104; 202; 212; 222; 300) of the catheter (102) and protrude distally beyond the distal end (104; 202; 212; 222; 300) of the catheter (102), the puncturing device (106; 201; 211; 221; 400) having a stiffness sufficient to straighten the distal end (104; 202; 212; 222; 300) of the catheter (102) when the puncturing device (106; 201; 211; 221; 400) is extended distally from the distal end (104; 202; 212; 222; 300) of the catheter (102), wherein the access device includes a first electrode formed on one of the distal end (104; 202; 212; 222; 300) of the catheter (102) and a distal end of the puncturing device (106; 201; 211; 221; 400), the first electrode being configured to apply energy to tissue contacting one of the distal end (104; 202; 212; 222; 300) of the catheter (102) and the distal end of the puncturing device (106; 201; 211; 221; 400).

2. The access device of claim 1, wherein the distal end (104; 202; 212; 222; 300) of the catheter (102) is rotatable about a longitudinal axis of the catheter (102) to direct a distal opening of the distal end (104; 202; 212; 222; 300) in a desired direction within the target tissue.

3. The access device of any one of claims 1-2, wherein the puncturing device (106; 201; 211; 221; 400) is coupled to the catheter (102) so that, when the access device punctures the target tissue, the distal end (104; 202; 212; 222; 300) of the catheter (102) is advanceable together with the puncturing device (106; 201; 211; 221; 400) into the target tissue.

4. The access device of any one of claims 1-3, wherein the first electrode is formed on the distal end of the catheter (102).

5. The access device of claim 4, wherein a proximal portion of the catheter (102) between the distal end of the catheter (102) and a distal end of a handle is formed from a non-conductive material,
further comprising a conducting wire connecting the first electrode to an actuator accessible to a user.

6. The access device any one of claims 1-5, further comprising:
a second electrode formed at the distal end of the puncturing device (106; 201; 211; 221; 400) configured to apply energy to tissue contacting the distal end of the puncturing device (106; 201; 211; 221; 400).

7. The access device of claim 6, further comprising:
an insulation layer electrically insulating the catheter (102) from the puncturing device (106; 201; 211; 221; 400).

8. The access device of any one of claims 1-7, further comprising:
an electrosurgical sheath longitudinally slidable along an exterior of the catheter (102), the electrosurgical sheath having an electrosurgical tip configured to dilate an access hole created by the access device in the target tissue.

9. The access device of any one of claims 1-8, further comprising a handle (500) including:
a length adjust (506);
a puncture actuator (508) configured to advance the puncturing device (106; 201; 211; 221; 400) and the catheter (102) distally out of a sheath (110) of the access device;
an electrosurgical sled (514) slidably mounted over the puncture actuator (508) to advance the sheath (110) distally over the catheter (102) to apply energy to the target tissue via the electrosurgical tip of the sheath (110);
a puncture actuator lock (510);
an electrosurgical sled lock (518) to lock a position of the sheath (110) in a desired position relative to the catheter (102); and
a first generator connection (516) on the electrosurgical sled (514) configured to couple to a source of electric energy.

10. The access device of claim 9, wherein the handle further includes a second generator connection (522) on the puncture actuator (508) configured to couple to the source of electric energy.

11. The access device of claim 9 or 10, further comprising:
a third generator connection (520) on the handle (500) configured to couple to a source of electric energy, the third generator connection (520) being electrically coupled to the second electrode.

12. The access device of any one of claims 1-11, wherein the distal end has an exposed metal end.

13. The access device of any one of claims 1-12, wherein the puncturing device (106; 201; 211; 221; 400) has a pointed tip.

14. The access device of any one of claims 1-13, wherein the access device is configured such that, when the puncturing device (106; 201; 211; 221; 400) is withdrawn proximally out of the distal end, the distal end is freed to return to its curved J-shape.

15. The access device of any one of claims 1-14, wherein distal end of the catheter (102) is straightened by the stiffness of the puncturing device until the puncturing device (106; 201; 211; 221; 400) extends distally out the distal end by a desired distance.

## Patentansprüche

1. Zugangsvorrichtung (100; 200; 210; 220), aufweisend:
einen Katheter (102), der ein sich dadurch erstreckendes Lumen aufweist, wobei der Katheter so bemessen und geformt ist, um sich durch einen endoskopischen Schaft zu einem Zielgewebe innerhalb eines lebenden Körpers zu erstrecken, wobei der Katheter (102) ein distales Ende (104; 202; 212; 222; 300) aufweist, das vorgespannt ist, relativ zu einem Abschnitt des Katheters (102), der sich von dem distalen Ende (104; 202; 212; 222; 300) in proximaler Richtung erstreckt, eine J-förmige Krümmung anzunehmen, wenn das distale Ende (104; 202; 212; 222; 300) nicht eingeschränkt ist, wobei das distale Ende (104; 202; 212; 222; 300) stumpf ist; und
eine Punktionsvorrichtung (106; 201; 211; 221; 400), die so bemessen und geformt ist, um sich durch das Lumen des Katheters (102) zu erstrecken, und dazu eingerichtet ist, sich in distaler Richtung aus dem distalen Ende (104; 202; 212; 222; 300) des Katheters (102) zu erstrecken und in distaler Richtung über das distale Ende (104; 202; 212; 222; 300) des Katheters (102) hinaus vorzustehen, wobei die Punktionsvorrichtung (106; 201; 211; 221; 400) eine Steifigkeit aufweist, die ausreicht, um das distale Ende (104; 202; 212; 222; 300) des Katheters (102) zu begradigen, wenn sich die Punktionsvorrichtung (106; 201; 211; 221; 400) in distaler Richtung aus dem distalen Ende (104; 202; 212; 222; 300) des Katheters (102) erstreckt, wobei die Zugangsvorrichtung eine erste Elektrode aufweist, die entweder an dem distalen Ende (104; 202; 212; 222; 300) des Katheters (102) oder an einem distalen Ende der Punktionsvorrichtung (106; 201; 211; 221; 400) ausgebildet ist, wobei die erste Elektrode dazu eingerichtet ist, Energie auf Gewebe aufzubringen, das entweder mit dem distalen Ende (104; 202; 212; 222; 300) des Katheters (102) oder mit dem distalen Ende der Punktionsvorrichtung (106; 201; 211; 221; 400) in Kontakt steht.

2. Zugangsvorrichtung nach Anspruch 1, wobei das distale Ende (104; 202; 212; 222; 300) des Katheters (102) um eine Längsachse des Katheters (102) drehbar ist, um eine distale Öffnung des distalen Endes (104; 202; 212; 222; 300) in eine gewünschte Richtung innerhalb des Zielgewebes auszurichten.

3. Zugangsvorrichtung nach einem der Ansprüche 1 bis 2, wobei die Punktionsvorrichtung (106; 201; 211; 221; 400) derart mit dem Katheter (102) gekoppelt ist, dass, wenn die Zugangsvorrichtung das Zielgewebe punktiert, das distale Ende (104; 202; 212; 222; 300) des Katheters (102) zusammen mit der Punktionsvorrichtung (106; 201; 211; 221; 400) in das Zielgewebe vorschiebbar ist.

4. Zugangsvorrichtung nach einem der Ansprüche 1 bis 3, wobei die erste Elektrode an dem distalen Ende des Katheters (102) ausgebildet ist.

5. Zugangsvorrichtung nach Anspruch 4, wobei ein proximaler Abschnitt des Katheters (102) zwischen dem distalen Ende des Katheters (102) und einem distalen Ende eines Handgriffs aus einem nichtleitenden Material gebildet ist, wobei die Zugangsvorrichtung ferner einen leitenden Draht aufweist, der die erste Elektrode mit einem für einen Benutzer zugänglichen Aktuator verbindet.

6. Zugangsvorrichtung nach einem der Ansprüche 1 bis 5, ferner aufweisend:
eine zweite Elektrode, die an dem distalen Ende der Punktionsvorrichtung (106; 201; 211; 221; 400) ausgebildet und dazu eingerichtet ist, Energie auf Gewebe aufzubringen, das mit dem distalen Ende der Punktionsvorrichtung (106; 201; 211; 221; 400) in Kontakt steht.

7. Zugangsvorrichtung nach Anspruch 6, ferner aufweisend:
eine Isolierschicht, die den Katheter (102) elektrisch von der Punktionsvorrichtung (106; 201; 211; 221; 400) isoliert.

8. Zugangsvorrichtung nach einem der Ansprüche 1 bis 7, ferner aufweisend:
eine elektrochirurgische Hülse, die entlang einer Außenseite des Katheters (102) in Längsrichtung verschiebbar ist, wobei die elektrochirurgische Hülse eine elektrochirurgische Spitze aufweist, die dazu eingerichtet ist, eine durch die Zugangsvorrichtung in dem Zielgewebe erzeugte Zugangsöffnung aufzuweiten.

9. Zugangsvorrichtung nach einem der Ansprüche 1 bis 8, ferner aufweisend einen Handgriff (500), der aufweist:
eine Längenverstellung (506);
einen Punktionsaktuator (508), der dazu eingerichtet ist, die Punktionsvorrichtung (106; 201; 211; 221; 400) und den Katheter (102) in distaler Richtung aus einer Hülse (110) der Zugangsvorrichtung heraus vorzuschieben;
einen elektrochirurgischen Schlitten (514), der verschiebbar auf dem Punktionsaktuator (508) montiert ist, um die Hülse (110) in distaler Richtung über den Katheter (102) vorzuschieben, um über die elektrochirurgische Spitze der Hülse (110) Energie auf das Zielgewebe aufzubringen;
eine Verriegelung (510) des Punktionsaktuators;
eine Verriegelung (518) des elektrochirurgischen Schlittens, um eine Position der Hülse (110) in einer gewünschten Position relativ zu dem Katheter (102) zu verriegeln; und
einen ersten Generatoranschluss (516) an dem elektrochirurgischen Schlitten (514), der dazu eingerichtet ist, mit einer Quelle elektrischer Energie gekoppelt zu werden.

10. Zugangsvorrichtung nach Anspruch 9, wobei der Handgriff ferner einen zweiten Generatoranschluss (522) an dem Punktionsaktuator (508) aufweist, der dazu eingerichtet ist, mit der Quelle elektrischer Energie gekoppelt zu werden.

11. Zugangsvorrichtung nach Anspruch 9 oder 10, ferner aufweisend:
einen dritten Generatoranschluss (520) an dem Handgriff (500), der dazu eingerichtet ist, mit einer Quelle elektrischer Energie gekoppelt zu werden, wobei der dritte Generatoranschluss (520) elektrisch mit der zweiten Elektrode gekoppelt ist.

12. Zugangsvorrichtung nach einem der Ansprüche 1 bis 11, wobei das distale Ende ein freiliegendes Metallende aufweist.

13. Zugangsvorrichtung nach einem der Ansprüche 1 bis 12, wobei die Punktionsvorrichtung (106; 201; 211; 221; 400) eine spitz zulaufende Spitze aufweist.

14. Zugangsvorrichtung nach einem der Ansprüche 1 bis 13, wobei die Zugangsvorrichtung derart eingerichtet ist, dass, wenn die Punktionsvorrichtung (106; 201; 211; 221; 400) in proximaler Richtung aus dem distalen Ende herausgezogen wird, das distale Ende freigegeben wird, um zu seiner gekrümmten J-Form zurückzukehren.

15. Zugangsvorrichtung nach einem der Ansprüche 1 bis 14, wobei das distale Ende des Katheters (102) durch die Steifigkeit der Punktionsvorrichtung begradigt wird, bis sich die Punktionsvorrichtung (106; 201; 211; 221; 400) um eine gewünschte Strecke in distaler Richtung aus dem distalen Ende erstreckt.

## Revendications

1. Dispositif d'accès (100 ; 200 ; 210 ; 220) comprenant :
un cathéter (102) comprenant une lumière s'étendant à travers ce dernier, le cathéter étant dimensionné et formé pour s'étendre à travers une tige endoscopique jusqu'à un tissu cible dans un corps vivant, le cathéter (102) comprenant une extrémité distale (104 ; 202 ; 212 ; 222 ; 300) sollicitée pour adopter une courbe en forme de J par rapport à une partie du cathéter (102) s'étendant de manière proximale à partir de l'extrémité distale (104 ; 202 ; 212 ; 222 ; 300), lorsque l'extrémité distale (104 ; 202 ; 212 ; 222 ; 300) n'est pas contrainte, dans lequel l'extrémité distale (104 ; 202 ; 212 ; 222 ; 300) est émoussée ; et
un dispositif de perforation (106 ; 201 ; 211 ; 221 ; 400) dimensionné et formé pour s'étendre à travers la lumière du cathéter (102) et configuré pour s'étendre de manière distale à partir de l'extrémité distale (104 ; 202 ; 212 ; 222 ; 300) du cathéter (102) et faire saillie, de manière distale, au-delà de l'extrémité distale (104 ; 202 ; 212 ; 222 ; 300) du cathéter (102), le dispositif de perforation (106 ; 201 ; 211 ; 221 ; 400) ayant une rigidité suffisante pour redresser l'extrémité distale (104 ; 202 ; 212 ; 222 ; 300) du cathéter (102) lorsque le dispositif de perforation (106 ; 201 ; 211 ; 221 ; 400) est étendu de manière distale à partir de l'extrémité distale (104 ; 202 ; 212 ; 222 ; 300) du cathéter (102), dans lequel le dispositif d'accès comprend une première électrode formée sur l'une parmi l'extrémité distale (104 ; 202 ; 212 ; 222 ; 300) du cathéter (102) et une extrémité distale du dispositif de perforation (106 ; 201 ; 211 ; 221 ; 400), la première électrode étant configurée pour appliquer de l'énergie sur le tissu qui est en contact avec l'une parmi l'extrémité distale (104 ; 202 ; 212 ; 222 ; 300) du cathéter (102) et l'extrémité distale du dispositif de perforation (106 ; 201 ; 211 ; 221 ; 400).

2. Dispositif d'accès selon la revendication 1, dans lequel l'extrémité distale (104 ; 202 ; 212 ; 222 ; 300) du cathéter (102) peut tourner autour d'un axe longitudinal du cathéter (102) afin de diriger une ouverture distale de l'extrémité distale (104 ; 202 ; 212 ; 222 ; 300) dans une direction souhaitée à l'intérieur du tissu cible.

3. Dispositif d'accès selon l'une quelconque des revendications 1 à 2, dans lequel le dispositif de perforation (106 ; 201 ; 211 ; 221 ; 400) est couplé au cathéter (102) de sorte que, lorsque le dispositif d'accès perfore le tissu cible, l'extrémité distale (104 ; 202 ; 212 ; 222 ; 300) du cathéter (102) peut avancer conjointement avec le dispositif de perforation (106 ; 201 ; 211 ; 221 ; 400) dans le tissu cible.

4. Dispositif d'accès selon l'une quelconque des revendications 1 à 3, dans lequel la première électrode est formée sur l'extrémité distale du cathéter (102).

5. Dispositif d'accès selon la revendication 4, dans lequel une partie proximale du cathéter (102) entre l'extrémité distale du cathéter (102) et une extrémité distale d'une poignée est formée à partir d'un matériau non conducteur,
comprenant en outre un fil conducteur raccordant la première électrode à un actionneur accessible à un utilisateur.

6. Dispositif d'accès selon l'une quelconque des revendications 1 à 5, comprenant en outre :
une deuxième électrode formée au niveau de l'extrémité distale du dispositif de perforation (106 ; 201 ; 211 ; 221 ; 400) configurée pour appliquer de l'énergie sur le tissu en contact avec l'extrémité distale du dispositif de perforation (106 ; 201 ; 211 ; 221 ; 400).

7. Dispositif d'accès selon la revendication 6, comprenant en outre :
une couche d'isolation isolant électriquement le cathéter (102) du dispositif de perforation (106 ; 201 ; 211 ; 221 ; 400).

8. Dispositif d'accès selon l'une quelconque des revendications 1 à 7, comprenant en outre :
une gaine électrochirurgicale pouvant coulisser de manière longitudinale le long d'un extérieur du cathéter (102), la gaine électrochirurgicale ayant une pointe électrochirurgicale configurée pour dilater un trou d'accès créé par le dispositif d'accès dans le tissu cible.

9. Dispositif d'accès selon l'une quelconque des revendications 1 à 8, comprenant en outre une poignée (500) comprenant :
un dispositif de réglage de longueur (506) ;
un actionneur de perforation (508) configuré pour faire avancer le dispositif de perforation (106 ; 201 ; 211 ; 221 ; 400) et le cathéter (102) de manière distale hors d'une gaine (110) du dispositif d'accès ;
un chariot électrochirurgical (514) monté, de manière coulissante, sur l'actionneur de perforation (508) afin de faire avancer la gaine (110) de manière distale sur le cathéter (102) pour appliquer l'énergie sur le tissu cible via la pointe électrochirurgicale de la gaine (110) ;
un verrou d'actionneur de perforation (510) ;
un verrou de chariot électrochirurgical (518) pour verrouiller une position de la gaine (110) dans une position souhaitée par rapport au cathéter (102) ; et
un premier raccordement de générateur (516) sur le chariot électrochirurgical (514) configuré pour se coupler à une source d'énergie électrique.

10. Dispositif d'accès selon la revendication 9, dans lequel la poignée comprend en outre un deuxième raccordement de générateur (522) sur l'actionneur de perforation (508) configuré pour se coupler à la source d'énergie électrique.

11. Dispositif d'accès selon la revendication 9 ou 10, comprenant en outre :
un troisième raccordement de générateur (520) sur la poignée (500) configuré pour se coupler à une source d'énergie électrique, le troisième raccordement de générateur (520) étant électriquement couplé à la deuxième électrode.

12. Dispositif d'accès selon l'une quelconque des revendications 1 à 11, dans lequel l'extrémité distale a une extrémité métallique exposée.

13. Dispositif d'accès selon l'une quelconque des revendications 1 à 12, dans lequel le dispositif de perforation (106 ; 201 ; 211 ; 221 ; 400) a une pointe pointue.

14. Dispositif d'accès selon l'une quelconque des revendications 1 à 13, dans lequel le dispositif d'accès est configuré de sorte que, lorsque le dispositif de perforation (106 ; 201 ; 211 ; 221 ; 400) est retiré de manière proximale hors de l'extrémité distale, l'extrémité distale est libérée pour revenir à sa forme de J courbée.

15. Dispositif d'accès selon l'une quelconque des revendications 1 à 14, dans lequel l'extrémité distale du cathéter (102) est redressée par la rigidité du dispositif de perforation jusqu'à ce que le dispositif de perforation (106 ; 201 ; 211 ; 221 ; 400) s'étende de manière distale à partir de l'extrémité distale sur une distance souhaitée.
